# FASCICULE DE BREVET EUROPEEN

(11) **EP 3 508 195 B1**
(45) Date de publication et mention de la délivrance du brevet: **05.04.2023**
(21) Numéro de dépôt: 19150107.1
(22) Date de dépôt: 02.01.2019
(51) Int. Cl.: A61K 8/9789, A61Q 19/00, A61P 17/04, A61K 36/537

(54) **EXTRAITS DE RACINES DE SALVIA MILTIORRHIZA ET LEUR UTILISATION TOPIQUE POUR APPAISER LA PEAU**
EXTRAKTE AUS DEN WURZELN VON SALVIA MILTIORRHIZA SOWIE DEREN VERWENDUNG IN TOPISCHEN ZUBEREITUNGEN ZUR BERUHIGUNG DER HAUT
SALVIA MILTIORRHIZA ROOT EXTRACTS AND THEIR USE IN TOPICAL COMPOSITIONS FOR SOOTHING SKIN

(30) Priorité: 05.01.2018 FR 1800012
(43) Date de publication de la demande: 10.07.2019
(73) Titulaire: Societe Industrielle Limousine d'Application Biologique, 19130 Objat (FR)
(72) Inventeur: PAUFIQUE, Jean, 19130 OBJAT (FR)
(74) Mandataire: Aquinov

(56) Documents cités:
- EP-A1- 1 679 058
- WO-A1-2016/055737
- CN-A- 105 267 175
- CN-A- 106 177 408
- CN-B- 102 580 054
- YANG Q ET AL: "Skin anti-allergic traditional Chinese medicinal composition useful for e.g. treating skin inflammation, and itching, comprises e.g. alkanna-root extract, Forsythia extract, radix salviae miltiorrhizae root extract, and mint extract", WPI / 2017 CLARIVATE ANALYTICS,, vol. 2017, no. 16, 7 décembre 2016 (2016-12-07), XP002782219,
- SUN Y ET AL: "Isolation and purification of salvianolic acid A and salvianolic acid B from Salvia miltiorrhiza by high-speed counter-current chromatography and comparison of their antioxidant activity", JOURNAL OF CHROMATOGRAPHY B: BIOMEDICAL SCIENCES & APPLICATIONS, ELSEVIER, AMSTERDAM, NL, vol. 877, no. 8-9, 15 mars 2009 (2009-03-15), pages 733-737, XP026002102, ISSN: 1570-0232, DOI: 10.1016/J.JCHROMB.2009.02.013 [extrait le 2009-02-11]
- CHEN J ET AL: "Separation and identification of water-soluble salvianolic acids from Salvia miltiorrhiza Bunge by high-speed counter-current chromatography and ESI-MS analysis", TALANTA, ELSEVIER, AMSTERDAM, NL, vol. 69, no. 1, 15 mars 2006 (2006-03-15), pages 172-179, XP025000665, ISSN: 0039-9140, DOI: 10.1016/J.TALANTA.2005.09.041 [extrait le 2006-03-15]
- LEE SE-EUN ET AL: "Anti-inflammatory effects of Salviae Miltiorrhizae Radix extract on RAW264.7 cell. via anti-oxidative activities", THE KOREA ASSOCIATION OF HERBOLOGY, KOREAN INTELLECTUAL PROPERTY OFFICE, vol. 30, no. 4, 30 juillet 2015 (2015-07-30), pages 89-94, XP053032863, ISSN: 1229-1765

## Description

L'invention concerne le traitement cosmétique des peaux agressées par des stimuli extérieurs tels que le froid, la pollution et les stress chimiques. L'invention se rapporte en particulier à un extrait naturel issu de végétaux, à des compositions cosmétiques l'incluant et à son utilisation cosmétique en application topique pour apaiser les peaux agressées.

Le syndrome des peaux sensibles a pris une ampleur considérable ces vingt dernières années. En effet, aujourd'hui, un nombre de plus en plus important de personnes présente une peau sensible ou intolérante.

La sensibilité cutanée est un désordre courant dont les symptômes sont des picotements, des échauffements, des tiraillements et/ou des rougeurs qui engendrent des conséquences psychologiques non négligeables. Différents paramètres contribuent au déclenchement de ces symptômes :
- D'une part des facteurs génétiques, en lien avec le sexe ou l'ethnie : les femmes sont plus touchées que les hommes en raison d'un épiderme plus fin et de changements hormonaux; les peaux asiatiques présentent une susceptibilité plus importante aux agressions que les peaux caucasiennes ou afro-américaines
- D'autre part le mode de vie : la peau sensible réagit anormalement à des agressions de différentes natures :
   o Environnementales (UV, chaleur, froid, pollution)
   o Chimiques (détergents, eau calcaire)
   o Physiologiques (stress, hormones)
   o Physiques (vêtements, rasage, épilation).

D'un point de vue biologique, les peaux sensibles ou intolérantes présentent un dysfonctionnement de trois composantes biologiques :
- une hyperactivité neuronale, c'est-à-dire une réponse excessive des terminaisons nerveuses, en particulier une hyperstimulation de nocicepteurs dont TRPV1 qui est le récepteur de référence,
- une fonction barrière altérée, en particulier une modification de la composition lipidique et du comportement des kératinocytes, et
- une inflammation exacerbée, en particulier une production en excès de substances pro-inflammatoires.

Parmi les facteurs environnementaux, le froid et la pollution figurent parmi les stress majeurs ayant le plus d'impact sur les peaux sensibles. En effet, ils perturbent l'équilibre cutané en agissant directement sur les trois composantes biologiques des peaux sensibles, décuplant ainsi les sensations désagréables au niveau de la peau.

Or, actuellement, les traitements cosmétiques proposés pour apaiser les peaux sensibles sont majoritairement focalisés sur des problèmes de sensibilité cutanée à des agressions chimiques, mais sans prendre en compte l'environnement, le mode de vie ou l'ethnie des personnes si bien que leur action n'est pas satisfaisante. En outre, ils n'agissent pas sur l'ensemble des trois composantes biologiques des peaux sensibles.

L'objectif de l'invention est de proposer une solution qui pallie ces inconvénients, en particulier une solution d'origine naturelle qui présente une action transversale sur les trois composantes biologiques impliquées dans la sensibilité cutanée, et qui présente une efficacité apaisante « multi-ethnique » et adaptée au mode de vie et à l'environnement des personnes concernées.

A cet effet, l'invention a pour objet l'utilisation d'un extrait de racines de *Salvia miltiorrhiza* pour un traitement topique cosmétique apaisant de la peau.

*Salvia miltiorrhiza* ou sauge rouge est connue également sous le nom de *Danshen.* Il s'agit d'une plante médicinale cultivée en Chine, mais est également très présente à l'état sauvage. Dotée d'une grande capacité de résistance aux stress abiotiques, elle s'adapte à tout changement d'environnement. En effet, la majorité des cultures de *Salvia miltiorrhiza* sont situées dans les régions de Sichuan et de Shandong où les hivers sont rudes avec de basses températures. Cultivée pour ses vertus thérapeutiques à la fois sur le corps et sur l'esprit, la racine de *Salvia miltiorrhiza* séchée est un incontournable de la pharmacopée chinoise car elle est un véritable concentré de molécules dites "bonne santé". Utilisée traditionnellement depuis des millénaires, la racine de sauge rouge est encore très présente dans les pratiques de médecine moderne. Parmi les propriétés décrites dans la médecine traditionnelle chinoise, *Danshen* serait capable d'améliorer la circulation sanguine, de soigner les maladies cardiovasculaires, cérébrales, hépatiques ou encore quelques maladies cutanées.

Des mélanges d'extraits sont ainsi déjà connus, en particulier à visée anti-inflammatoire tel que décrit par Yang Q et al. 2016 ou dans la demande de brevet CN 106177408 ou CN105267175, mais également pour des produits sous forme de shampoing tel que décrit dans CN102580054. Un mélange de plantes dans le traitement de l'angiocardiopathie est également décrit dans la demande EP1679058.

La demande WO2016055737 nous enseigne la combinaison d'un extrait de *Salvia miltiorrhiza* et de niacine et/ou de niacimide et leur activité de déglycation. Lee Se-Eun et al. 2015, décrit des molécules anti-inflammatoires dans les racines de *Salvia miltiorrhiza.* Or, lesdits mélanges ou lesdits de l'art antérieur ne comprennent pas au moins 5% de polyphénols et ne sont donc pas pertinents pour répondre au problème technique de la présente invention.

Enfin, Sun Y et al. 2009 nous enseigne un procédé de purification de *Salvia miltiorrhiza* mis en oeuvre avec de l'éthanol et Chen J et al. nous enseigne un procédé de purification d'acide salvianoliques mis en oeuvre avec de l'eau. Il n'est donc pas décrit un procédé d'extraction selon l'invention et par conséquent l'extrait selon l'invention.

Ains, l'invention vise l'utilisation d'un extrait de *Danshen* en cosmétique pour un effet spécifique apaisant, et non de *Danshen* tel que visée dans la pharmacopée chinoise ou japonaise.

Avantageusement un tel extrait :
- est obtenu par la mise en oeuvre d'un procédé d'extraction à l'aide de solvants d'origine végétale,
- présente une action sur les trois composantes biologiques impliquées dans la sensibilité cutanée :
   o diminution de l'hyperactivité neuronale, en particulier inactivation du récepteur TRPV1,
   o effet bénéfique sur la fonction barrière et amélioration de la barrière cutanée
   o diminution de l'inflammation,
- présente un effet apaisant de la peau, avec une efficacité sur les peaux caucasiennes ou asiatiques sujettes à l'hypersensibilité,
- constitue une solution globale et adaptée au mode de vie et à l'environnement des personnes concernées en présentant une action apaisante de la peau soumise au froid ou à la pollution.

Ainsi l'utilisation d'un extrait de *Salvia miltiorrhoza* selon l'invention permet d'apaiser la peau et d'apporter confort et protection aux peaux délicates, en particulier au niveau du visage et des mains. L'invention permet également avantageusement de nourrir et d'hydrater durablement la peau, de la rendre plus douce, plus résistante aux agressions extérieures, d'atténuer les rougeurs de la peau, de la rendre moins sensible, moins irritable, moins inconfortable.

L'extrait selon l'invention est un extrait spécifique de racines de *Salvia miltiorrhiza* comprenant au moins 5% en poids d'acides phénoliques par rapport au poids total de matière sèche de l'extrait. L'invention vise également des compositions cosmétiques contenant un tel extrait.

L'invention a aussi pour objet un procédé de traitement cosmétique, non thérapeutique, de la peau pour un effet apaisant consistant en l'application topique sur la peau d'une composition comprenant l'extrait de racines de *Salvia miltiorrhiza.* D'autres caractéristiques et avantages de l'invention, ressortiront de la description en détails de l'invention qui va suivre en regard des figures qui représentent :
- Figure 1 : le chromatogramme HPLC du standard de Cryptotanshinone 0.25g/L,

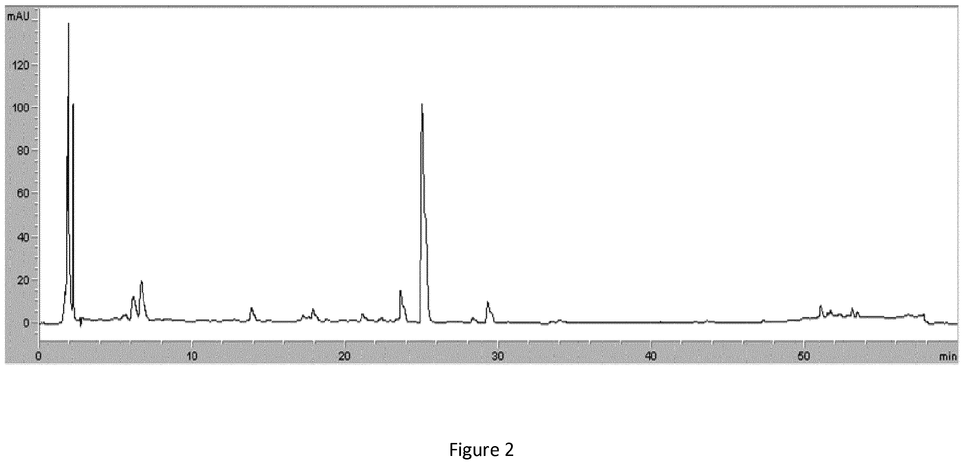

- Figure 2 : le chromatogramme HPLC de l'extrait de l'exemple 1 selon l'invention,
- Figure 3 : résultat de chromatographie sur couche mince de racine de *Salvia Miltiorrhiza* et de cryptotanshinone (lumière blanche)
- Figure 4: résultat de chromatographie sur couche mince d'un extrait selon l'invention et de cryptotanshinone (lumière blanche)

### DEFINITIONS

Par « agent cosmétique » au sens de l'invention, on entend également un principe actif cosmétique ou un agent actif cosmétique, c'est à dire au moins une molécule, préférentiellement un ensemble de plusieurs molécules présentant un effet sur les cellules de la peau.

Par « contenant un « X » » ou « comprenant un « X ». », sauf indication contraire, au sens de l'invention on entend qui contient ou comprend au moins un « X ». « X » est quantifiable selon une méthode analytique choisie en fonction de « X » et des connaissances de l'homme du métier.

Par « extrait » de racines de *Salvia miltiorrhiza* au sens de l'invention on entend toute molécule ou mélange de molécules obtenue(s) à partir de racines *Salvia miltiorrhiza.* Il peut s'agir d'une ou plusieurs molécule(s) native(s) ou d'une ou plusieurs molécule(s) obtenue(s) par tout type d'extraction et transformation des molécules natives de racines de *Salvia miltiorrhiza.*

Par « peau sensible » ou « peau intolérante » au sens de l'invention on entend une peau présentant un seuil de tolérance diminué par rapport aux picotements, échauffements, tiraillements, rougeurs, etc.

### DESCRIPTION DETAILLEE DE L'INVENTION

L'invention a donc pour objet l'utilisation d'un extrait de racines de *Salvia miltiorrhiza* pour un traitement topique cosmétique apaisant de la peau. Par traitement cosmétique on entend cosmétique non thérapeutique.

En effet, selon l'invention un extrait de racines de *Salvia miltiorrhiza* lorsqu'il est appliqué sur la peau, en particulier sur une peau sensible, est capable de l'apaiser, et notamment :
- de neutraliser l'hyperactivité neuronale de la peau, et/ou
- de préserver la fonction barrière de la peau, et/ou
- de limiter l'inflammation de la peau.

Préférentiellement l'invention vise l'utilisation d'un extrait de racines de *Salvia miltiorrhiza* pour agir sur ces trois facteurs à la fois.

Ainsi l'invention vise un extrait de racines de *Salvia miltiorrhiza* pour son utilisation dans le traitement topique apaisant de la peau, en particulier pour :
- de neutraliser l'hyperactivité neuronale de la peau, et/ou
- de préserver la fonction barrière de la peau, et/ou
- de limiter l'inflammation de la peau.

L'hyperactivité neuronale est une réponse excessive des terminaisons nerveuses de la peau liée à une hyperactivation des nocicepteurs. En effet, la peau héberge un réseau de fibres nerveuses impliquées dans la sensorialité, c'est-à-dire la perception des sensations de douleur, de démangeaisons et les thermo-sensations. Cette fonction est assurée par une famille de récepteurs nommée TRP (Transient receptor potential). Au sein de cette famille, TRPV1 (Transient receptor potential vanilloid 1) joue un rôle crucial. En effet, ce récepteur est transversal, il peut être activé par une grande variété de stimuli exogènes et endogènes, dont certains agonistes naturels comme la capsaïcine. Les études récentes suggèrent que les réactions cosmétiques hypersensibles cutanées sont étroitement liées à l'innervation de l'épiderme et à l'hyper activation de TRPV1. Ce dernier est d'ailleurs aujourd'hui considéré comme le marqueur de référence des peaux sensibles (Duarte et al., 2017; Gouin et al., 2017). Au niveau des neurones sensitifs, son activation conduit à des influx de calcium et à la libération de neuromédiateurs responsables d'une inflammation neurogène cutanée (Boillat et al., 2014; Gouin et al., 2017). Egalement exprimé par d'autres types cellulaires tels que les kératinocytes, l'activation du TRPV1 induit leur apoptose ainsi que la synthèse et la sécrétion de substances pro-inflammatoires. Ces phénomènes limitent ainsi l'établissement d'une fonction barrière fonctionnelle (Tóth et al., 2014; Misery et al., 2016)

TRPV1 occupe donc une place centrale dans la sensibilité cutanée. Son activation est responsable de l'apparition d'un lot de sensations cutanées déplaisantes telles que douleurs, démangeaisons, échauffements, picotements (Misery et al., 2016; Duarte et al., 2017). Son inactivation constitue donc un pré-requis pour le développement d'un actif apaisant.

Selon l'invention, l'utilisation d'un extrait de racines de *Salvia miltiorrhiza* sur la peau, en particulier sur une peau sensible, permet d'inhiber l'activation du récepteur des peaux sensibles TRPV1 et limite ainsi l'hyperréactivité neuronale cutanée et les manifestations cutanées qui en découlent tels que des picotements.

En plus de l'hyperréactivité neuronale, de nombreuses études suggèrent que la sensibilité cutanée est liée à une altération de la fonction barrière. Cette modification est à l'origine d'une sensation d'inconfort (Misery et al., 2016). Dans les peaux sensibles, il a été mis en évidence :
- une perte du film hydrolipidique protecteur ;
- une modification de l'organisation et de la composition lipidiques du *stratum corneum ;*
- une faible adhésion cornéocytaire (Draelos, 1997; Tóth et al., 2014; Misery et al., 2016; Richters et al., 2017).

A ceci s'ajoute l'apoptose des kératinocytes induite par l'influx de calcium généré par l'activation de TRPV1 (Tóth et al., 2014).

L'endommagement de la barrière cutanée conduit à :
- une augmentation de la perte insensible en eau expliquant la sécheresse ressentie par les personnes sujettes à l'hypersensibilité cutanée (Saint-Martory et al., 2008; Pinto et al., 2011; Duarte et al., 2017; Richters et al., 2017) ;
- une exposition accrue des neurones sensitifs et par conséquent des perceptions sensorielles exacerbées (Richters et al., 2015) ;
- une pénétration facilitée de substances irritantes et allergisantes qui sont à l'origine d'une inflammation caractérisée par la production de cytokines, leucotriènes et prostaglandines (Draelos, 1997; Misery et al., 2016; Duarte et al., 2017)

Dans les peaux sensibles, l'altération de la fonction barrière entraîne une déshydratation qui participe à l'apparition de sensations de tiraillements, d'irritations et d'inconfort. Préserver la fonction barrière constitue donc un élément important qui conditionne le confort apporté par un soin.

Selon l'invention, l'utilisation d'un extrait de racines de *Salvia miltiorrhiza* sur la peau, en particulier sur une peau sensible, permet de diminuer significativement la perte insensible en eau et de booster significativement l'hydratation de la peau, notamment au niveau du visage et des mains.

L'hyperréactivité neuronale et l'altération de la fonction barrière entraînent le déclenchement de processus pro-inflammatoires :
- au niveau des neurones sensitifs, les neuromédiateurs sécrétés en réponse à la stimulation de TRPV1 activent les kératinocytes localisés à proximité des terminaisons nerveuses (Tóth et al., 2014; Misery et al., 2016; Gouin et al., 2017). En se fixant sur leurs récepteurs spécifiques, ils induisent la synthèse de substances pro-inflammatoires telles que la cyclooxygénase 2 (COX-2), la prostaglandine E2 (PGE2), les leucotriènes B4 (LTB4).
- suite à l'altération de la fonction barrière, la pénétration des substances irritantes et allergisantes provoque une inflammation caractérisée par la production de cytokines, prostaglandines et leucotriènes (Duarte et al., 2017).

La production de ce cocktail inflammatoire induit :
- le recrutement de cellules inflammatoires renforçant l'importance et la chronicité de l'inflammation (Tóth et al., 2014; Misery et al., 2016; Gouin et al., 2017) ;
- l'activation indirecte de TRPV1, entretenant ainsi l'hyperréactivité neuronale (Gouin et al., 2017) ;
- des modifications fonctionnelles de la barrière cutanée (Vestergaard et al., 2012).

Dans les peaux sensibles, l'inflammation chronique conduit à des démangeaisons et des rougeurs. Maîtriser le phénomène inflammatoire représente donc une action importante pour apaiser les peaux sensibles.

Selon l'invention, l'utilisation d'un extrait de racines de *Salvia miltiorrhiza* sur la peau, en particulier sur une peau sensible, permet d'inhiber significativement la sécrétion de PGE2 et la synthèse de COX dans des peaux agressées. Un extrait de racines de *Salvia miltiorrhiza* est par conséquent capable de limiter la réponse inflammatoire de la peau face à une agression, et permet ainsi de réduire les rougeurs cutanées des peaux agressées.

L'utilisation d'un extrait de racines de *Salvia miltiorrhiza* sur la peau permet donc d'améliorer la réactivité neuronale, la fonction barrière et l'état inflammatoire de la peau, et par conséquent aide les peaux sensibles dans leur lutte contre les stimuli du quotidien. Le confort de la peau est amélioré, les tiraillements et les rougeurs diminués et la peau est apaisée, plus douce, plus hydratée, plus résistante aux agressions extérieures. L'utilisation d'un extrait de racines de *Salvia miltiorrhiza* sur la peau permet de rendre la peau moins sensible, moins irritable, moins inconfortable et hydratée durablement.

L'invention a donc spécifiquement pour objet, l'utilisation cosmétique d'un extrait de racines de *Salvia miltiorrhiza* dans une composition cosmétique en tant qu'agent cosmétique, en particulier en tant qu'agent cosmétique destiné à apaiser la peau, notamment pour :
- neutraliser l'hyperréactivité neuronale de la peau, et/ou
- préserver la fonction barrière de la peau, et/ou
- limiter l'inflammation de la peau,
et spécifiquement pour réduire les réactions cosmétiques hypersensibles cutanées et/ou diminuer les sensations de tiraillement de la peau.

L'invention vise une utilisation sur toutes les peaux, en particulier pour les peaux sensibles et encore plus particulièrement pour les peaux ayant subi une agression :
o environnementale (UV, chaleur, froid, pollution), et/ou
o chimique (détergents, eau calcaire), et/ou
o physiologique (stress, hormones) et/ou
o physique (vêtements, rasage, épilation).

Préférentiellement, l'invention vise l'utilisation d'un extrait de racines de *Salvia miltiorrhiza* pour un traitement topique cosmétique non thérapeutique apaisant des peaux agressées par le froid et/ou par la pollution et/ou par des stress chimiques.

L'extrait de racines de *Salvia miltiorrhiza* agit avantageusement à la fois sur les peaux caucasiennes, et asiatiques.

Les extraits de racines de *Salvia miltiorrhiza* selon l'invention sont des extraits de racines de *Salvia miltiorrhiza* comprenant au moins 5% en poids de polyphénols totaux par rapport au poids total de matière sèche de l'extrait, préférentiellement une teneur entre 5 et 15% de polyphénols totaux par rapport au poids total de matière sèche de l'extrait.

La teneur en polyphénols totaux peut être mesurée par exemple par dosage colorimétrique. Encore plus préférentiellement, il s'agit d'un extrait comprenant au moins 70% de polyphénols de type acides salvianoliques en poids par rapport au poids total des polyphénols totaux présents dans l'extrait. Les acides salvianoliques sont une famille d'acides phénoliques constitués de plusieurs molécules. On peut citer l'acide salvianolique A, l'acide savianolique B, l'acide salvianolique C, l'acide methylsalvianolique C, l'acide isosalvianolique C, l'acide salvianique C, l'acide salvianolique E, l'acide salvianolique F, l'acide salvianolique I, l'acide salvianolique J. Le terme « acides salvianoliques » ou « de type acides salvianoliques » signifie donc au moins un de ces acides salvianoliques.

Préférentiellement les acides de type acides salvianoliques représentent au moins 3,5% en poids de matière sèche de l'extrait.

La caractérisation des acides phénoliques présents dans l'extrait est réalisée par exemple par chromatographie liquide couplée à la spectrométrie de masse.

Les polyphénols totaux et en particulier les acides phénoliques de type acides salvianoliques, participent activement à l'efficacité de l'extrait pour apaiser la peau, et notamment présentent une action importante sur la sécrétion de PGE2.

De façon préférée l'extrait de racines de *Salvia miltiorrhiza* selon l'invention ne comprend pas de cryptotanshinone contrairement à la racine de la plante qui en contient. Or cette molécule présente un danger pour risque de cardiotoxicité et malformation du développement.

La méthode d'identification de cryptotanshinone utilisée pour démontrer son absence dans l'extrait selon l'invention est soit celle décrite dans la Pharmacopée Japonaise pour l'identification de la racine de *Salvia miltiorrhiza,* soit par chromatographie liquide avec détection UV.

Selon un mode de réalisation particulièrement adapté, l'extrait selon l'invention, en plus des polyphénols, comprend :
- des sucres, et/ou
- des protéines, et/ou
- des minéraux.

La teneur en sucres dans l'extrait peut être déterminée par la méthode de DUBOIS (Dubois M. et al., Analytical chemistry, 28, 3, 350-356, 1956).

Les extraits selon l'invention comprennent donc une quantité importante de carbohydrates. Préférentiellement, la teneur en sucres de l'extrait selon l'invention est d'au moins 75% en poids de matière sèche de l'extrait.

De façon préférée, les carbohydrates de l'extrait présentent les caractéristiques suivantes :
- les carbohydrates contenus dans l'extrait sont des sucres de masse molaire inférieure à 1260 Da, et/ou
- au moins 60% des carbohydrates sont des oligosaccharides et des polysaccharides de masses molaires comprises entre 180 et 1260Da (degré de polymérisation compris entre 1 et 7), le pourcentage étant donné en poids par rapport au poids total des carbohydrates de l'extrait,
- moins de 40% des carbohydrates sont des monosaccharides, le pourcentage étant donné en poids par rapport au poids total des carbohydrates de l'extrait.

Les sucres simples constituant les carbohydrates de l'extrait selon l'invention, sont préférentiellement le galactose, le fructose et le glucose.

Concernant les protéines, les extraits selon l'invention ont préférentiellement une teneur en protéines inférieure à 5% en poids par rapport au poids total de matière sèche de l'extrait. La teneur en protéines est préférentiellement déterminée par la méthode de KJELDHAL (référence : Official method of analysis of the A.O.C., 12th ed. W Horwitz, E.D., New-York, 15-60, 1975).

Concernant les minéraux, les extraits selon l'invention ont préférentiellement une teneur en cendres minérales inférieure à 8% en poids par rapport au poids total de matière sèche de l'extrait.

La teneur en cendres brutes peut être déterminée par la pesée des résidus issus de l'incinération des échantillons de l'extrait à 550°C dans un four à moufle électrique.

Un extrait de racines de *Salvia miltiorrhiza* particulièrement adapté est un extrait obtenu à l'aide d'un solvant d'origine naturelle, en particulier un solvant d'origine végétale tel qu'un mélange d'eau / butylène glycol d'origine végétale.

L'extrait selon l'invention peut se présenter sous forme solide ou sous forme liquide, préférentiellement sous forme liquide.

Lorsqu'il se présente sous forme liquide, il se présente de façon préférée sous forme d'un liquide limpide avec une couleur jaune.

L'extrait selon l'invention peut être obtenu par tout procédé permettant d'obtenir un extrait de racines de *Salvia miltiorrhiza* présentant les caractéristiques revendiquées. Préférentiellement il s'agit d'un procédé comprenant au moins une étape de solubilisation de racines de de *Salvia miltiorrhiza* et au moins une étape de tri moléculaire.

L'extrait de racines de *Salvia miltiorrhiza* selon l'invention peut notamment être obtenu par un procédé comprenant la mise en oeuvre des étapes suivantes :
- solubilisation de racines de *Salvia miltiorrhiza* entre 50 et 500g/l, dans un mélange eau/butylène glycol (ratio eau/butylène glycol compris entre 30/90 et 5/95, par exemple à raison de 15/85),
- séparation des phases soluble et insoluble, préférentiellement par décantation, par filtration, par centrifugation,
- récupération de la phase soluble
- filtration avec tri moléculaire pour éliminer les molécules ayant une masse molaire supérieure à 2000Da,
- filtration stérilisante

L'extrait selon l'invention est préférentiellement utilisé comme agent cosmétique dans des compositions, ces compositions comprenant un milieu cosmétiquement acceptable. Il s'agit de compositions dans différentes formes galéniques, adaptées à une application sur la peau, en particulier la peau du visage.

Ces compositions peuvent se présenter notamment sous forme d'émulsions huile-dans-eau, émulsions eau-dans-huile, émulsions multiples (Eau/Huile/Eau ou Huile/Eau/Huile) qui peuvent être éventuellement des microémulsions ou des nanoémulsions, ou sous forme de solutions, suspensions, hydrodispersions, crèmes, gels, gels aqueux ou poudres. Elles peuvent être plus ou moins fluides et avoir l'aspect de lotion, shampoing, crème ou mousse. Préférentiellement il s'agit de crèmes ou de gels.

Il peut s'agir de compositions comprenant au moins 0,25% d'un extrait de racines de *Salvia miltiorrhiza* selon l'invention, préférentiellement entre 0,5 et 5%, les pourcentages étant donnés en poids par rapport au poids de la composition.

Ces compositions comprennent, outre l'extrait de racines de *Salvia miltiorrhiza,* un milieu physiologiquement acceptable et de préférence cosmétiquement acceptable, c'est-à-dire qui ne provoque pas de sensations d'inconfort inacceptables pour l'utilisateur telles que des rougeurs, tiraillements ou picotements.

Les compositions selon l'invention peuvent contenir comme adjuvant au moins un composé choisi parmi :
- les huiles, qui peuvent être choisies notamment parmi les huiles de silicone, linéaires ou cycliques, volatiles ou non volatiles ;
- les cires, telles que l'ozokérite, la cire de polyéthylène, la cire d'abeille ou la cire de carnauba,
- les élastomères de silicone,
- les tensioactifs, de préférence émulsionnants, qu'ils soient non ioniques, anioniques, cationiques ou amphotères,
- les co-tensioactifs, tels que les alcools gras linéaires,
- les épaississants et/ou gélifiants,
- les humectants, tels que les polyols comme la glycérine,
- les colorants, les conservateurs, les charges,
- les tenseurs,
- les séquestrants,
- les parfums,
- et leurs mélanges, sans que cette liste soit limitative.

Des exemples de tels adjuvants sont cités notamment dans le Dictionnaire CTFA *(International Cosmetic Ingrédient Dictionary and Handbook* publié par le *Personal Care Product Council*). Bien entendu, l'homme du métier veillera à choisir les éventuels composés complémentaires, actifs ou non-actifs, et leur quantité, de telle sorte que les propriétés avantageuses du mélange ne soient pas, ou sensiblement pas, altérées par l'adjonction envisagée.

Ces compositions sont notamment destinées à être utilisées sur la peau pour un effet apaisant.

L'invention a par conséquent également pour objet un procédé cosmétique de traitement, non thérapeutique, de la peau pour un effet apaisant, en particulier pour un effet apaisant des peaux sensibles, consistant en l'application topique sur la peau d'une composition comprenant un extrait selon l'invention. Le procédé est particulièrement adapté pour un effet apaisant des peaux agressées par le froid et/ou par la pollution et/ou par des stress chimiques. L'invention vise par conséquent des compositions selon l'invention pour une utilisation pour une application topique sur la peau pour un effet apaisant, en particulier des peaux sensibles, et notamment des peaux agressées par le froid et/ou la pollution et/ou par des stress chimiques.

Afin d'illustrer les effets cosmétiques d'un extrait de racines de *Salvia miltiorrhiza,* les exemples suivants avec leurs résultats d'essais sont présentés.

### EXEMPLES

### Exemples d'extraits

Plusieurs extraits selon l'invention ont été réalisés en faisant varier les paramètres du procédé. L'étude analytique des différents extraits présentés dans les exemples a été réalisée comme suit :
- La matière sèche est effectuée en pesant 3 g de produit ajouté à 10 g de sable placés à l'étuve à 105°C pendant 40 h.
- Le pH est mesuré par la méthode potentiométrique. Les mesures sont effectuées à température ambiante.
- L'azote total est dosé selon la méthode de KJELDHAL (référence : Official method of analysis of the A.O.C., 12th ed. W Horwitz, E.D., New-York, 15-60, 1975). Cette méthode permet de déterminer la teneur en protéines.
- La teneur en cendres brutes est déterminée par la pesée des résidus issus de l'incinération des échantillons des extraits selon l'invention à 550°C dans un four à moufle électrique (VULCAN^{™} 3.550-NDI).
   5 grammes d'échantillon sont pesés dans un creuset préalablement taré et sont placés dans le four.
   Le programme de minéralisation comprend un premier palier de 3 heures à 110°C suivi d'un deuxième palier de 9 heures à 550°C. La température de 550°C est maintenue jusqu'à ce que les cendres soient blanches. Le creuset et son contenu sont ensuite placés immédiatement dans un dessiccateur jusqu'à complet refroidissement et pesés. Le poids du résidu est calculé en déduisant la tare.
- Les sucres totaux sont dosés à l'aide de la méthode de DUBOIS (Dubois M. et al., Analytical chemistry, 28, 3, 350-356, 1956).
   Toutes les fonctions réductrices sont libérées en présence d'acide sulfurique concentré et donnent avec le phénol un composé coloré jaune-orangé. La coloration obtenue, mesurée à 490 nm sur un spectrophotomètre, est proportionnelle à la quantité de sucres totaux de l'échantillon.
   Des solutions de calibration sont préparées à partir d'un standard de glucose entre 25 à 125 mg/L. Une courbe de calibration des densités optiques des solutions de calibration en fonction de leur concentration est construite.
   Les échantillons des extraits selon l'invention sont préalablement dilués avec de l'eau distillée, pour que la teneur en sucres corresponde à la gamme d'étalonnage.
   La quantité de sucres totaux des échantillons est déterminée grâce à la courbe de calibration.
- La quantification des polyphénols est réalisée par dosage colorimétrique.
   Les composés phénoliques forment en présence de ferricyanure de potassium et de chlorure de fer des complexes colorés qui peuvent être dosés par spectrophotométrie à 715 nm. L'intensité de cette coloration est proportionnelle à la quantité de composés phénoliques présents dans l'échantillon.
   Des solutions de calibration sont préparées à partir d'un standard d'hespéridine de 40 à 120 mg/L. Une courbe de calibration des densités optiques des solutions de calibration en fonction de leur concentration est construite.
   Les échantillons des extraits sont préalablement dilués avec de l'eau distillée, pour que la teneur en polyphénols corresponde à la gamme d'étalonnage.
   La quantité de polyphénols des échantillons est déterminée grâce à la courbe de calibration.

### Exemple 1 d'extrait selon l'invention

L'extrait de l'exemple 1 est obtenu par la mise en oeuvre du procédé suivant :
- solubilisation de 50g de poudre de racines séchées de *Salvia miltiorrhiza* dans 1l d'un mélange eau/butylène glycol (ratio eau/butylène glycol 10/90),
- séparation des phase soluble et insoluble, par décantation,
- récupération de la phase soluble
- filtration avec tri moléculaire pour éliminer les molécules ayant une masse molaire supérieure à 2000Da,
- filtration stérilisante sur 0.22µ.

L'extrait de l'exemple 1 se présente sous forme d'un liquide limpide de couleur jaune. Le taux de matière sèche est de 29.2g/l.

La composition chimique de l'extrait de l'exemple 1 est la suivante (les pourcentages étant donnés en poids de matière sèche) :
- Sucres : 25.9g/l, soit 89%
- Polyphénols : 2.2g/l en gamme hespéridine, soit 8%,
- Cendres : 2.5%
- Protéines : 0.5%

Les composés phénoliques de l'extrait sont répartis comme suit :
- Acides phénoliques hydrophiles : 85%
- Autres composés : 15%

La teneur en acide salvianolique B est évaluée à 105ppm.

L'absence de cryoptotanshinone a été déterminée par chromatographie liquide avec détection UV, selon les conditions suivantes :
- Colonne: Alltech Altima C18 150 x 4.6 mm diam 3 µm avec une pré-colonne avec les mêmes caractéristiques.
- Solvants:
- A: Eau ultrapure avec 0.05% d'acide formique
- B: acetonitrile

- Gradient:

| Temps (minutes) | % A | % B |
|---|---|---|
| 0 | 95 | 5 |
| 45 | 40 | 60 |
| 50 | 0 | 100 |
| 55 | 0 | 100 |
| 60 | 95 | 5 |

- débit: 1 mL/min
- Température: 35°C
- Détection : UV détection à 280 nm
- Volume d'injection : 10 µL

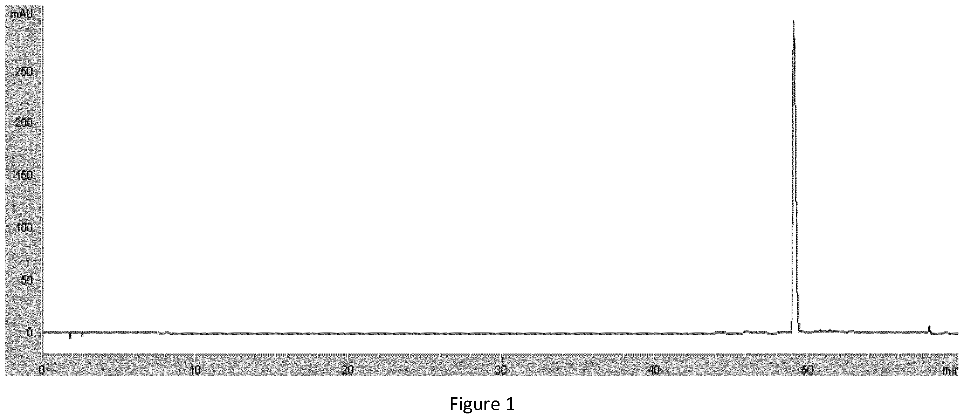

Le standard de cryptotanshinone à 0.25g/l présente un pic d'élution à 49mn (Figure 1). Il n'y a pas de pic d'élution pour l'extrait selon l'invention au temps de rétention de 49mn (cf Figue 2).

L'extrait selon l'invention ne contient pas de cryptotanshinone. La limite de quantification a été déterminée à 5ppm.

### Exemple 2

L'extrait de l'exemple 2 est obtenu par la mise en oeuvre du procédé suivant :
- solubilisation de 100g de poudre de racines séchées de *Salvia miltiorrhiza* dans 1l d'un mélange eau/butylène glycol (ratio eau/butylène glycol 20/80),
- séparation des phase soluble et insoluble, par décantation,
- récupération de la phase soluble
- décoloration par ajout d'adjuvant,
- filtration avec tri moléculaire pour éliminer les molécules ayant une masse molaire supérieure à 2000Da,
- filtration stérilisante sur 0.22µ.

L'extrait de l'exemple 2 se présente sous forme d'un liquide limpide de couleur jaune. Le taux de matière sèche est de 30.0g/l.

La composition chimique de l'extrait de l'exemple 2 est la suivante (les pourcentages étant donnés en poids de matière sèche) :
- Sucres : 25.2g/l, soit 84%
- Polyphénols : 3.3 g/l, soit 11%
- Cendres : 4%
- Protéines : 1%

La teneur en acide salvianolique B est évaluée à 392ppm.

L'absence de cryptotanshinone a été montrée au moyen de la méthode décrite dans la monographie de « Salvia miltiorrhiza Root » de la Pharmacopée Japonaise.

La monographie décrit la méthode d'identification suivante :
Placer 1g de composé Salvia miltiorrhiza root, dans 10ml de diethyl ether. Agiter pendant 10mn et filtrer. Evaporer le filtrat et dissoudre le résidu dans 1ml d'éthyl acétate. Utiliser cette solution comme l'échantillon. Déposer l'échantillon sur une Thin-layer chromatography sous forme d'un spot de 10µl. développer la plaque de silice avec un mélange hexane et ethyl acetate (3 :1) pendant environ 10cm. Sécher la plaque. Un sport rouge marron au temps Rf de 0,4 est observé.

La chromatographie sur couche mince a été réalisée :
1/ sur la racine de Salvia miltiorrhiza
2/ sur une solution standard de la cryptotanshinone (Sigma C5624-98%) de2 g/l dans du méthanol
3/ sur le principe actif selon l'invention de l'exemple 2 à différentes concentrations
4/ sur le principe actif selon l'invention de l'exemple 2 après filtration sur C18-Maxi-Clean ^{™} cartridge
5/ sur une solution standard de cryptotanshinone de 1 g/l après filtration sur C18-Maxi-Clean ^{™} cartridge.

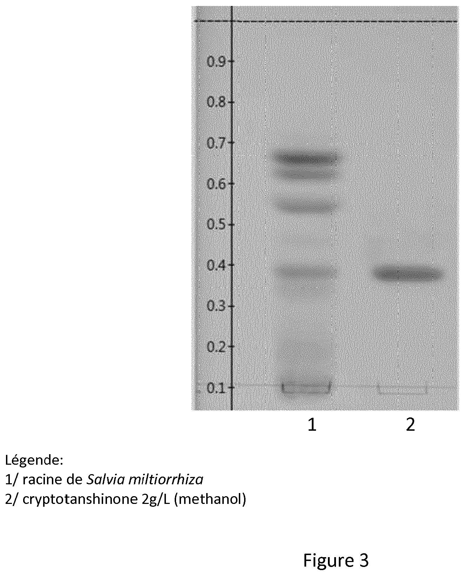

La cryptotanshinone est le composé ayant un Rf de 0,4 dans les conditions de l'étude, décrite par la pharmacopée Japonaise. L'échantillon 1, la racine de Salvia miltiorrhiza, montre bien la présence de cryptotanshinone dans la plante (Figure 3).

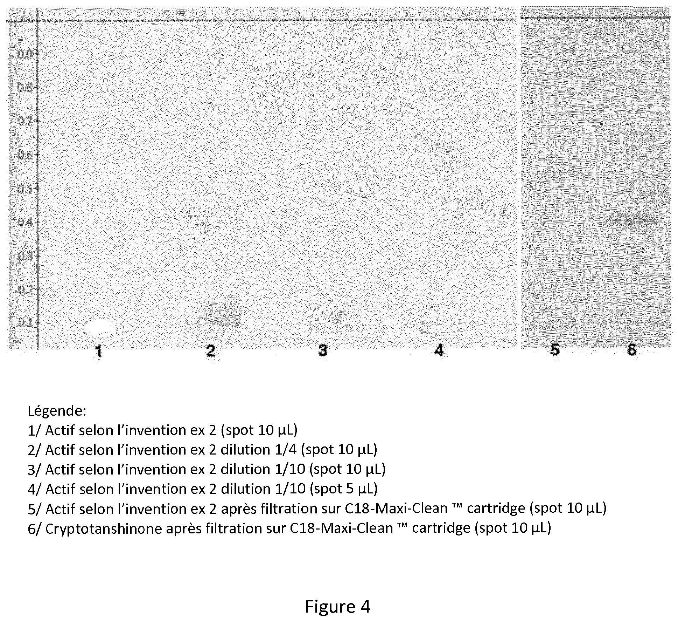

Par contre, l'extrait selon l'invention a été analysé conformément cette méthodologie. Le résultat est présenté sur la Figure 4. Les profils 1 à 4 présentent une interférence entre le solvant de l'extrait et le solvant d'élution. Pour cela, une pré-filtration a été réalisée au moyen d'une C18-Maxi-Clean ^{™} cartridge pour éliminer le solvant de l'extrait avant dépôt (profil 5). Cet essai a aussi été réalisé sur une solution standard de cryptotanshinone à 1g/l dans le même solvant (profil 6).

La molécule de cryptotanshinone n'est pas présente dans l'extrait selon l'invention (profil 5), et elle est toujours présente dans la solution standard (profil 6).

L'extrait selon l'invention ne contient pas de cryptotanshinone.

### Exemples compositions

### Exemple 3 : Formule anti-rougeur comprenant un extrait de racines de Salvia miltiorrhiza

La formulation est la suivante :

| | | | |
|---|---|---|---|
| A. | Eau | | qsp 100% |
| | Conservateur | | 1% |
| | Glycerine | | 2% |
| | extrait de l'exemple 1 | | **2,5%** |
| | | | |
| B. | DUB 1632 | (Stéarinerie Dubois) | 1% |
| | Lanol1688 | (Seppic) | 5% |
| | DUB 5545 | (Stéarinerie Dubois) | 5% |
| | Myglyol 812N | (Cremer Oleo GmbH & Co. KG) | 4% |
| | Tegosoft TN2 | (Evonik Industries AG Personal Care) | 1% |
| | | | |
| C. | Carbopol EDT 2020 | (Lubrizol) | 1% |
| | | | |
| D. | NaOH | | qsp pH= 6,1 |

Cette composition est un gel-émulsionné, légèrement écru, brillant, sans odeur, texture souple.

Elle présente une application aisée, un étalement doux et glissant, une pénétration rapide, un fini doux, léger et un effet filmogène.

Le pH de la composition est de 6,1.

Elle est obtenue par la mise en oeuvre des étapes suivantes :
- Mélanger A. Chauffer au bain-marie à 80°C sous agitation magnétique,
- Mélanger B. Chauffer au bain-marie à 80°C sous agitation magnétique,
- A 80°C, émulsionner A dans B sous rotor stator à 1800 tr/min,
- A 30°C, ajouter C, sous rotor stator à 1500 tr/min,
- Ajuster ensuite le pH, avec D, toujours sous agitation.

### Exemple 4 : Gel émulsionné comprenant un extrait de racines de Salvia miltiorrhiza

La formulation est la suivante :

| | | | |
|---|---|---|---|
| A. | Eau | | qsp 100% |
| | Conservateur | | 1% |
| | extrait de l'exemple 2 | | 2,5% |
| | | | |
| B. | DUB SSIC | (Stéarinerie Dubois) | 2,5% |
| | DUB ININ A | (Stéarinerie Dubois) | 2,5% |
| | DUB DIS | (Stéarinerie Dubois) | 1% |
| | | | |
| C. | DC 200 | (Dow Corning) | 5% |
| | Sepiplus 400 | (Seppic) | 1,5% |

Cette composition est une émulsion gélifiée, blanche, brillante, sans odeur, texture épaisse. Elle présente une préhension souple et légère, un étalement frais et glissant et une pénétration assez rapide, avec un fini doux et sec et un touché siliconé.

La composition a un pH de 5,2.

Elle est obtenue par la mise en oeuvre des étapes suivantes :
- Mélanger A. Chauffer au bain-marie à 50°C sous agitation magnétique, de façon à bien homogénéiser le conservateur.
- Ajouter B, et agiter sous rotor stator à 2000 tr/min.
- A 30°C, ajouter C, dans l'ordre indiqué, sous rotor stator à 1500 tr/min.
- Laisser sous agitation jusqu'à complète homogénéisation.

### Exemple 5 : Formule crème masque comprenant un extrait de racines de Salvia miltiorrhiza

La formulation est la suivante :

| | | | |
|---|---|---|---|
| A. | Eau | | qsp 100% |
| | Conservateur | | 1% |
| | | | |
| B. | DUB MM | (Stéarinerie Dubois) | 0,5% |
| | DUB Vinyl | (Stéarinerie Dubois) | 0,5% |
| | Phytowax Olive 16L55 | (Sophim) | 0,5% |
| | Montanov 14 | (Seppic) | 3% |
| | Sensanov WR | (Seppic) | 2% |
| | Miroir De Sucre | (Laboratoire Hytech) | 3% |
| | Euthanol G 16 S | (BASF) | 5% |
| | | | |
| C. | Sepimax ZEN | (Seppic) | 0,5% |
| | extrait de l'exemple 1 | | **2,5%** |
| | | | |
| D. | NaOH | | qsp pH= 5,9 |

La composition est une émulsion blanche, brillante, sans odeur, avec une texture semi-épaisse.

Elle présente une application souple, un excellent étalement à sensorialité rafraîchissante typique de l'effet « quick break », et un fini filmogène, gras et brillant, permettant un temps de pause avant le retrait de l'excédent.

La composition a un pH de 5,9.

Elle est obtenue par la mise en oeuvre des étapes suivantes :
- Mélanger A. Chauffer au bain-marie à 80°C sous agitation magnétique, en veillant à bien disperser le conservateur.
- Mélanger B. Chauffer au bain-marie à 80°C sous agitation magnétique.
- Emulsionner A dans B, sous rotor stator à 1500 tr/min.
- Dès complète homogénéisation, ajouter C, et laisser agiter sous rotor stator à 1300 tr/min.
- A température ambiante, et sous agitation, ajuster le pH avec D.
- Laisser quelques minutes sous rotor-stator à 1000 tr/min.

### Exemple 6 : Formule crème de jour comprenant un extrait de racines de Salvia miltiorrhiza

La formulation est la suivante :

| | | | |
|---|---|---|---|
| A. | Eau | | qsp 100% |
| | Conservateur | | 1% |
| | extrait de l'exemple 2 | | **2,5%** |
| | | | |
| B. | Beurre de Karité | (Sophim) | 2% |
| | Beurre de Pêche | (Sophim) | 1% |
| | DUB Dipa | (Stéarinerie Dubois) | 5% |
| | Sophiderm | (Sophim) | 5% |
| | Easynov | (Seppic) | 3% |
| | Montanov L | (Seppic) | 3% |
| | DUB CG7 | (Stéarinerie Dubois) | 2% |
| | Beurre de Cupuaçu | (Laboratoire Hytech) | 2% |
| | | | |
| C. | DUB Velvet Gum | (Stéarinerie Dubois) | 4% |
| | DC 9040 | (Dow Corning) | 4% |

Cette composition est une émulsion siliconée, blanche, brillante, sans odeur, texture ferme. Elle présente une application douce, un étalement glissant et un effet évanescent, avec une pénétration assez rapide et un fini doux et filmogène.

La composition a un pH 5,3.

Elle est obtenue par la mise en oeuvre des étapes suivantes :
- Mélanger A. Chauffer au bain-marie à 80°C sous agitation magnétique.
- Mélanger B. Chauffer au bain-marie à 80°C sous agitation magnétique.
- Emulsionner B dans A, sous rotor stator à 2200 tr/min.
- A froid, ajouter C, et agiter sous rotor stator à 2000 tr/min.
- Laisser sous agitation jusqu'à complète.

### EVALUATION DE L'EFFICACITE COSMETIQUE SELON L'INVENTION

### 1. CAPACITE D'UN EXTRAIT SELON L'INVENTION A INHIBER LES RECEPTEURS TRPV1

L'objectif de cette étude est d'évaluer la capacité d'un extrait de racines de *Salvia miltiorrhiza,* à inhiber l'activité des récepteurs TRPV1 *(Transient receptor potential vanilloid 1)* des neurones sensitifs humains qui jouent un rôle majeur dans la détection et la transmission des sensations de douleur et de démangeaisons jusqu'au système nerveux central.

Cette étude a été réalisée sur un modèle de neurones sensitifs humains dérivés de cellules hiPS (human induced Pluripotent StemCells) en co-culture avec des kératinocytes humains. L'activation des récepteurs TRPV1 se traduit par une entrée massive de calcium dans le cytoplasme des neurones sensitifs qui peut être captée par une sonde fluorescente et analysée par microscopie à épifluorescence.

Le protocole opératoire est décrit en suivant :
- J0, culture des neurones sensitifs issus de cellules hiPS :
   Les cellules hiPS *(human induced Pluripotent Stem cells*) sont ensemencées dans du milieu de culture et incubées à 37°C.
- J14, mise en co-culture avec des kératinocytes humains normaux :
   Des kératinocytes humains sont ensemencés sur le tapis de neurones sensitifs. Les cellules sont ensuite incubées à 37°C dans une atmosphère contenant 5% de CO₂.
- J19 :
   ∘ Pré-traitement,
      La co-culture de neurones sensitifs/kératinocytes humains est prétraitée ou non avec un extrait selon l'invention de l'exemple à 0,25% (V/V) ou avec de la capsazepine à 10µM (inhibiteur de TRPV1).
   ∘ Incubation avec la sonde fluorescente Fluo-4,
      Le milieu de culture est éliminé et remplacé par un milieu contenant la sonde fluorescente Fluo-4 (Fluo-4 Acetoxymetyl) en présence ou non de l'extrait selon l'invention à 0,25% (V/V) ou de la capsazépine à 10µM.
   ∘ Traitement (activation des récepteurs TRPV1),
      A la fin de cette incubation, les co-cultures sont placées sous un microscope à épifluorescence. Les neurones sensitifs sont observés durant trois minutes au cours desquelles une image est prise toutes les 333 ms. Cinq secondes après le démarrage de l'enregistrement, les neurones sont stimulés avec de la capsaïcine à 10µM pour activer les récepteurs TRPV1. Le traitement à la capsaïcine est réalisé en présence ou non de l'extrait de l'exemple 1 à 0,25% (V/V) ou de capsazepine à 10µM.
   ∘ Analyse de l'activité des récepteurs TRPV1 par mobilisation de calcium cytoplasmique,
   L'activation des récepteurs TRPV1 induit une entrée de calcium dans les neurones sensitifs. Cette entrée de calcium est captée par la sonde Fluo-4 entraînant une augmentation du niveau de fluorescence dans les neurones. Plus l'augmentation du niveau de fluorescence est importante plus les récepteurs TRPV1 sont activés.

La moyenne de l'augmentation du niveau de fluorescence a été comparée en pourcentage par rapport à la condition contrôle non activée.

Les résultats sont présentés dans le Tableau 1 :

**Tableau 1. Capacité d'un extrait de racines de Salvia miltiorrhiza à inhiber l'activité des récepteurs TRPV1 de neurones sensitifs activés par la capsaïcine.**

| | **Mobilisation de calcium cytoplasmique (%)** | **Inhibition des récepteurs TRPV1 / neurones témoins activés (%)** |
|---|---|---|
| **Neurones sensitifs non activés** | | |
| Témoin | 100 | |

| **Neurones sensitifs activés par la capsaïcine** | | |
|---|---|---|
| Témoin | 330^{✧✧✧} | |
| Capsazépine 10µM | 131*** | 199% |
| Exemple selon l'invention exemple 1 à 0,25% | 215*** | 115% |

| | | |
|---|---|---|
| ^{✧✧✧} *: résultat significatif selon le test One Way ANOVA*/*neurones sensitifs témoins non activés (p <* 0,001) *** : *résultats significatifs selon le test One Way ANOVA*/*neurones sensitifs témoins activés (p <* 0,001) | | |

Le traitement de la co-culture par la capsaïcine entraîne une augmentation significative de la mobilisation du calcium cytoplasmique dans les neurones sensitifs.

Cette augmentation est significativement inhibée de 199% par un traitement à la capsazépine démontrant une activation spécifique des récepteurs TRPV1.

Testé à 0,25%, l'extrait selon l'invention inhibe significativement de 115% l'activation des récepteurs TRPV1 des neurones sensitifs. Il permet ainsi de limiter l'hyperréactivité des fibres nerveuses sensitives cutanées.

### II. CAPACITE D'UN EXTRAIT SELON L'INVENTION A LIMITER LA REPONSE INFLAMMATOIRE DANS UN CONTEXTE DE POLLUTION

### 11.1. Etude de la synthèse de COX-2

L'objectif de cette étude est d'évaluer la capacité d'un extrait de racines de *Salvia miltiorrhiza,* à limiter la synthèse de cyclo-oxygénase 2 (COX-2) lors d'une inflammation induite par un polluant (particules fines : PM).

Quotidiennement, la peau sensible est soumise à une multitude de stimuli extérieurs (rayonnements UV, pollution atmosphérique, etc...) pouvant engendrer une réaction inflammatoire non spécifique contribuant au sentiment d'inconfort. En réponse à un stress avec des particules fines (PM) les kératinocytes synthétisent de la COX-2, une enzyme ayant un rôle majeur dans le processus inflammatoire. Inhiber la synthèse de COX2 peut donc permettre de limiter les méfaits induits par la pollution.

L'expression de COX-2 a été évaluée par immunohistochimie sur des SILABSKIN^{®} RE soumis à des traitements répétés avec des particules fines (PM) afin de mimer un environnement pollué.

### A. Culture et traitements des SILABSKIN^{®} RE

Les kératinocytes humains sont cultivés dans un milieu de culture spécifique
- J0 :
   Les kératinocytes humains normaux sont ensemencés sur des inserts puis incubés à 37°C dans une atmosphère contenant 5% de CO₂.
- J2 à J13 :
   Le milieu de culture est changé tous les 2 jours.
- J14 et J15, traitements des *SILABSKIN*^{®} RE :
   Les *SILABSKIN*^{®} RE sont traités topiquement avec une solution de particules fines (PM) en présence ou non de l'extrait de l'exemple 1 à 0,25% (V/V) ou avec de la capsazépine à 1µM.
- J16 :
   Les *SILABSKIN*^{®} RE sont récupérés, fixés, déshydratés et inclus en paraffine. Des coupes (4 µm) sont ensuite réalisées à l'aide d'un microtome (RM2125RT, Leica).

### B. Analyse de l'expression de COX-2 par marquage immunohistochimique

- Déparaffinage en xylène
- Démasquage des sites antigéniques
- Saturation
- Anticorps primaire : anticorps monoclonal de lapin anti-COX-2
- Anticorps secondaire : anticorps anti-IgG de lapin couplé Alexa Fluor^{®} 488

L'expression de COX-2 est proportionnelle à l'intensité de la fluorescence verte présente sur les *SILABSKIN*^{®} RE. Une analyse quantitative des images a été réalisée à l'aide du logiciel Matlab^{®} version R2012b (MathWorks). Les résultats sont exprimés en unités arbitraires (UA). Les résultats obtenus sont donnés dans le Tableau 2.

**Tableau 2. Capacité d'un extrait selon l'invention à limiter la synthèse de COX-2 sur SILABSKIN^{®} RE lors d'une inflammation induite par un polluant (PM).**

| | **Synthèse de COX-2 (x10³ UA)** | **Synthèse de COX-2 / témoin pollué (%)** |
|---|---|---|
| **SILABSKIN^{®} RE normaux** | | |
| Témoin | 265 | |

| **SILABSKIN^{®} RE pollués** | | |
|---|---|---|
| Témoin | 899^{✧✧✧} | |
| Capsazépine 1 µM | 659* | -38 |
| Extrait selon l'invention exemple 1 à 0,25% | 682* | -34 |

| | | |
|---|---|---|
| ^{✧✧✧} : *résultat significatif selon le test de Wilcoxon Mann Withney* / *SILABSKIN*^{®} *RE témoins normaux (p <* 0,001) * : *résultats significatifs selon le test de Wilcoxon Mann Withney* / *SILABSKIN*^{®} *RE témoins pollués (p* < *0,05)* | | |

On constate que le traitement avec les particules fines (PM) induit une augmentation significative de l'expression de COX-2 sur les *SILABSKIN*^{®} RE.

Cette augmentation est significativement inhibée de 38% par un traitement à la capsazépine démontrant que la synthèse de COX-2 générée par les PM est en partie due à l'activation des récepteurs TRPV1.

Testée à 0,25% sur SILABSKIN^{®} RE, un extrait de racines de *Salvia miltiorrhiza,* inhibe significativement de 34% la synthèse de COX-2 induite par un traitement avec des particules fines (PM). Il permet ainsi de limiter la réaction inflammatoire générée par la pollution atmosphérique.

### 11.2. Etude de la sécrétion de PGE2

L'objectif de cette étude est d'évaluer la capacité d'un extrait de racines de *Salvia miltiorrhiza* à limiter la sécrétion de prostaglandines E2 (PGE2) lors d'une inflammation induite par un polluant (particules fines : PM).

Quotidiennement, la peau sensible est soumise à une multitude de stimuli extérieurs (rayonnements UV, pollution atmosphérique, etc...) pouvant engendrer une réaction inflammatoire non spécifique contribuant au sentiment d'inconfort. En réponse à un stress avec des particules fines (PM), les kératinocytes secrètent des PGE2 qui sont des médiateurs majeurs de l'inflammation. Inhiber la production de PGE2 peut donc permettre de limiter les méfaits de la pollution.

Cette étude a été réalisée par dosage ELISA sur le surnageant de culture de kératinocytes humains soumis à un traitement avec des particules fines (PM) mimant un environnement pollué.

Le protocole opératoire de l'étude est décrit en suivant.
- J0,

Les kératinocytes humains normaux sont ensemencés dans du milieu culture et incubés à 37°C dans une atmosphère contenant 5% de CO₂.
- J2 :
   - Pré-traitement :
      Les kératinocytes sont prétraités ou non avec l'extrait de l'exemple 1 à 0,025% et 0,05% (V/V) ou avec de la capsazépine à 0,5 µM (inhibiteur de TRPV1). Les cellules sont ensuite incubées pendant 30 minutes à 37°C dans une atmosphère contenant 5% de CO₂.
   - Traitement avec les particules fines (PM) :
      Les kératinocytes sont traités avec une solution de particules.
      Les cellules sont ensuite incubées pendant 24 heures à 37°C dans une atmosphère contenant 5% de CO₂.
- J3, récupération des surnageants :
   Les surnageants de culture sont récupérés et le dosage des PGE2 est réalisé à l'aide d'un kit ELISA (Enzo Life Sciences).

Les résultats obtenus sont donnés dans le Tableau 3.

**Tableau 3. Capacité d'un extrait selon l'invention à limiter la sécrétion de PGE2 par des kératinocytes humains lors d'une inflammation induite par un polluant (PM).**

| | **Taux de PGE2 (pg/mg de protéines)** | **Taux de PGE2 / témoin traité PM (%)** |
|---|---|---|
| **Kératinocytes normaux** | | |
| Témoin | 380 | |

| **Kératinocytes traités avec des PM** | | |
|---|---|---|
| Témoin | 627^{✧✧} | |
| Capsazépine 0,5 µM | 433* | -78 |
| Exemple selon l'invention exemple 1 à 0,025% | 512 | -47 |
| Exemple selon l'invention exemple 1 à 0,050% | 392* | -95 |

| | | |
|---|---|---|
| ✧✧ : *résultat significatif selon le test t de Student* / *kératinocytes témoins normaux (p <* 0,01) * : *résultats significatifs selon le test t de Student* / *kératinocytes témoins traités PM (p < 0,05)* | | |

On constate que le traitement avec les particules fines (PM) induit une augmentation significative de la sécrétion de PGE2 par les kératinocytes.

Cette augmentation est significativement inhibée de 78% par un traitement à la capsazépine démontrant que l'inflammation générée par les PM est en partie due à l'activation des récepteurs TRPV1 des kératinocytes.

Testée à 0,05%, un extrait de racines de *Salvia miltiorrhiza* augmente significativement de 95% la sécrétion de PGE2 par les kératinocytes suite à un traitement avec des particules fines (PM). Il permet ainsi de limiter fortement la réaction inflammatoire générée par la pollution atmosphérique.

### III. EFFET D'UN EXTRAIT SELON L'INVENTION SUR LA FONCTION BARRIERE

L'objectif de cette étude est d'évaluer *in vivo* l'influence de l'extrait de racines de *Salvia miltiorrhiza* formulé à 2,5% en émulsion sur la qualité de la fonction barrière au niveau du visage et des mains de personnes présentant une peau sensible.

Cet effet a été évalué à l'aide des méthodes suivantes :
- étude de la perte insensible en eau mesurée au Tewamètre^{®} ;
- étude du taux d'hydratation mesuré à l'aide d'un Cornéomètre^{®} ;
- auto-évaluation de la performance perçue via un questionnaire d'évaluation subjective.

La formule de la composition testée sur le visage est la suivante :

| | |
|---|---|
| Isononyl isononanoate (Lanol 99, Seppic) | 5,0% |
| Arachidyl alcohol / Behenyl Alcohol / Arachidyl glucoside (Montanov 202, Seppic) | 3,0% |
| *Principe actif selon l'invention (exemple 1)* | 2,5% |
| Cetearyl alcohol / cetearyl glucoside (Montanov 68, Seppic) | 2,0% |
| Conservateurs | 1,0% |
| Polyacrylamide / C13-14 isoparaffin / Laureth-7 (Sepigel 305, Seppic) | 0,3% |
| Eau | qsp 100 |

La formule de la composition testée sur les mains est la suivante :

| | |
|---|---|
| Glycérol | 5,0% |
| *Principe actif selon l'invention (exemple 1)* | 2,5% |
| PEG-7 glyceryl cocoate (DUB GC7, Dubois) | 1,2% |
| Conservateurs | 1,0% |
| Caprylic / capric triglycerides (DUB MCT 5545, Dubois) | 0,8% |
| Acrylate / C10-30 alkyl acrylate crosspolymer (Carbopol ULTREZ20, Noveon) | 0,3% |
| NaOH | Qsp pH 4,5 |
| Eau | qsp 100 |

Les études ont été réalisés avec les paramètres tels que décrits en suivants.

### A. Etude au niveau du visage

- Panel :
   20 volontaires sains caucasiens de sexe féminin, d'âge moyen 43 ± 10 ans ayant déclaré avoir une peau sensible selon les items du questionnaire SS-10 (Misery et al., Acta Derm Venereol, 2014), ayant réagi à la capsaïcine au cours d'un stinging test et présentant un seuil de tolérance au froid faible (mesure via le système TSA II).
- Modalités d'application :
   Applications biquotidiennes

### B. Etude au niveau des mains

- Panel :
   13 volontaires sains caucasiens de sexe féminin, d'âge moyen 46 ± 11 ans ayant déclaré avoir une peau sensible selon les items du questionnaire SCC-10 (Misery et al., Acta Derm Venereol, 2014) et présentant un seuil de tolérance au froid faible au niveau des mains (mesure via le système TSA II).
- Modalités d'application :
   4 applications par jour (matin 8h, midi, après-midi 16h, au coucher)

Les résultats obtenus pour les différents tests sont présentés en suivant.

### Perte insensible en eau :

Un résumé des résultats correspondant à l'effet d'un extrait de racines de *Salvia miltiorrhiza* formulé à 2,5% comparé au placebo, sur la perte insensible en eau de la peau mesurée à l'aide d'un Tewamètre^{®} au niveau du visage et des mains, est présenté dans le Tableau 4 :

**Tableau 4 Effet d'un extrait selon l'invention formulé à 2,5% sur la perte insensible en eau.**

| | **Variation/Placebo (%)** |
|---|---|
| Visage | -9,6 |
| Mains | -9,2 |

On constate qu'après 14 jours d'application, un extrait selon l'invention formulé à 2,5% diminue significativement la perte insensible en eau au niveau du visage et des mains (respectivement -9,6%, p=0,0012 et -9,2%, p=0,0287) chez des volontaires présentant une peau sensible. 70% d'entre eux ont présenté un effet positif sur le visage et 69% sur les mains.

### Etude du taux d'hydratation :

Un résumé des résultats correspondant à l'effet d'un extrait de racines de *Salvia miltiorrhiza* formulé à 2,5% comparé au placebo, sur l'hydratation cutanée mesurée à l'aide d'un Cornéomètre^{®} au niveau des joues et des mains, est présenté dans le Tableau 5.

**Tableau 5 Effet d'un extrait selon l'invention formulé à 2,5% sur le taux d'hydratation.**

| | **Variation/Placebo (%)** |
|---|---|
| Visage | +13,3 |
| Mains | +10,3 |

On constate qu'après 14 jours d'application, un extrait selon l'invention formulé à 2,5% augmente significativement le taux d'hydratation au niveau du visage et des mains (respectivement +13,2%, p=0,0001 et +10,3%, p=0,0147) chez des volontaires présentant une peau sensible. 90% d'entre eux ont présenté un effet positif sur le visage et 69% sur les mains.

### Evaluation subjective au niveau du visage :

Les résultats des questions fermées sont présentés dans le Tableau 6.

**Tableau 6. Auto-évaluation d'un extrait selon l'invention formulé à 2,5% après 14 jours d'applications biquotidiennes au niveau du visage en comparaison au placebo.**

| | **Cumul des réponses «plutôt d'accord» et «d'accord» (%)** | |
|---|---|---|
| | **Placebo** | **Extrait selon l'invention 2,5%** |
| **Peau plus résistante aux agressions extérieures** | 78 | 94 |
| **Peau hydratée durablement** | 67 | 83 |

On constate qu'après 14 jours d'application d'un extrait de racines de *Salvia miltiorrhiza,* la peau est :
- plus résistante face aux agressions extérieures pour 94% des volontaires contre 78% pour la formule placebo (différence significative, p = 0,0301)
- hydratée durablement pour 83% des volontaires contre 67% avec le placebo (p = 0,1131).

### Evaluation subjective au niveau des mains :

Les résultats des questions fermées sont présentés dans le Tableau 7.

**Tableau 7. Auto-évaluation d'un extrait de racines de Salvia miltiorrhiza formulé à 2,5% après 14 jours d'applications au niveau des mains en comparaison au placebo.**

| | **Cumul des réponses «plutôt d'accord» et «d'accord» (%)** | |
|---|---|---|
| | **Placebo** | **Extrait selon l'invention 2,5%** |
| **Mains plus hydratées** | 77 | 100 |
| **Mains plus douces** | 77 | 100 |
| **Mains moins sensibles aux agressions** | 62 | 92 |
| **Soin apaisant** | 46 | 92 |

On constate qu'après 14 jours d'application d'un extrait de racines de *Salvia miltiorrhiza* ^{®} au niveau des mains :
- l'ensemble des volontaires a constaté que leurs mains étaient plus hydratées et plus douces, contre 77% pour le placebo (respectivement p = 0,0003 et p = 0,0020) ;
- 92% d'entre elles indiquent que leurs mains semblent moins sensibles aux agressions, contre seulement 62% pour le placebo (p = 0,0043) ;
- 92% notent que le soin présente des propriétés apaisantes (p = 0,0002).

### IV EFFET D'UN EXTRAIT SELON L'INVENTION SUR LA SENSIBILITE CUTANEE

L'objectif de cette étude est d'évaluer *in vivo* l'effet d'un extrait de racines de *Salvia miltiorrhiza* formulé à 2,5% en émulsion sur la sensibilité cutanée au niveau du visage, avant et après 14 jours d'applications biquotidiennes. Cette étude a été réalisée sur volontaires caucasiens et sur volontaires asiatiques ayant un score de réactivité cutanée supérieur à 3 pour les peaux asiatiques, et 5 pour les peaux caucasiennes, lors de l'application de capsaïcine sur les ailes du nez.

Les études ont été réalisées avec les paramètres tels que décrits en suivants.

### A. Panel caucasien :

- Panel:
   20 volontaires sains de sexe féminin, d'âge moyen 43 ± 10 ans ayant déclaré avoir une peau sensible selon les items du questionnaire
   SS-10 (Misery et al., Acta Derm Venereol, 2014), ayant réagi à la capsaïcine au cours d'un stinging test et présentant un seuil de tolérance au froid faible (mesure via le système TSA II).
- Méthodes :
   étude de la réactivité cutanée après application de capsaïcine (3.10⁻⁴ %) (stinging test) ; et
   auto-évaluation de la performance perçue via un questionnaire d'évaluation subjective.

La formule cosmétiques testée sur les volontaires caucasiens est la suivante :

| | |
|---|---|
| Isononyl isononanoate (Lanol 99, Seppic) | 5,0% |
| Arachidyl alcohol / Behenyl Alcohol / Arachidyl glucoside (Montanov 202, Seppic) | 3,0% |
| *Principe actif selon l'invention (exemple 1)* | 2,5% |
| Cetearyl alcohol / cetearyl glucoside (Montanov 68, Seppic) | 2,0% |
| Conservateurs | 1,0% |
| Polyacrylamide / C13-14 isoparaffin / Laureth-7 (Sepigel 305, Seppic) | 0,3% |
| Eau | qsp 100 |

### B. Panel asiatique :

- Panel :
   31 volontaires sains de sexe féminin, d'âge moyen 44 ± 9 ans, ayant réagi à la capsaïcine au cours d'un stinging test et ayant été exposés à la pollution urbaine au moins pendant un an avant le démarrage de l'étude
- Méthodes :
   étude de la réactivité cutanée après application de capsaïcine (1.10⁻⁵ %) (stinging test) ; et
   étude de la neurosensibilité (Neurometer).

La formule cosmétique testée sur les volontaires asiatiques est la suivante :

| | |
|---|---|
| Glycérine | 6,2% |
| Cetearyl ethylhexanoate (Lanol 1688, Seppic) | 3,0% |
| Coco-Caprylate/ Caprate (DUB 810C, Dubois) | 3,0% |
| *Principe actif selon l'invention (exemple 1)* | 2,5% |
| Isononyl isononanoate (Lanol 99, Seppic) | 2,0% |
| Conservateurs | 1,0% |
| Polyacrylate Crosspolymer-6 (Sepimax zen, Seppic) | 0,5% |
| Hydroxyethyl Acrylate / Sodium Acryloyldimethyl Taurate Copolymer (Sepinov EMT 10, Seppic) | 0,5% |
| Aqua (water) | qsp100 |

Les résultats obtenus pour les différents tests sont présentés en suivant.

### Etude de la réactivité cutanée

Un résumé des résultats correspondant à l'effet d'un extrait de racines de *Salvia miltiorrhiza* formulé à 2,5% comparé au placebo, sur la réactivité cutanée évaluée à l'aide d'un stinging test, est présenté sur le Tableau 8 pour le panel caucasien et sur la Tableau 9 pour le panel asiatique.

**Tableau 8 Effet d'un extrait selon l'invention formulé à 2,5% sur les sensations de picotements - Panel caucasien**

| Score J14-J0 Perception des picotements | 0mn | 1mn | 2mn | 5mn |
|---|---|---|---|---|
| Placebo | 0.25 | -0.10 | -0.10 | -0.50 |
| Extrait selon l'invention 2,5% | -0.5* | -0.75** | -0.95** | -0.70* |

| | | | | |
|---|---|---|---|---|
| *** : différence significative* / *J0 (p < 0,01)* ** : différences significatives* / *J0 (p < 0,05)* | | | | |

**Tableau 9 Effet d'un extrait selon l'invention formulé à 2,5% sur les sensations de picotements - Panel asiatique**

| Score J14-J0 Perception des picotements | 0mn | 1mn | 2mn | 5mn |
|---|---|---|---|---|
| Placebo | -0.32 | -0.55 | -0.74 | -0.52 |
| Extrait selon l'invention 2,5% | -1.29* | -1.16 | -1.87** | -1.39* |

| | | | | |
|---|---|---|---|---|
| *** : différence significative* / *J0 (p < 0,01)* * : *différences significatives* / *J0 (p < 0,05)* | | | | |

On constate qu'après 14 jours d'application, un extrait de racines de *Salvia miltiorrhiza* améliore la tolérance de la peau à la capsaïcine sur un panel de volontaires caucasiens et asiatiques.

En effet, un extrait de racines de *Salvia miltiorrhiza* formulé à 2,5% limite significativement les sensations désagréables induites par l'application de capsaïcine sur les sillons naso-géniens.

### Etude de la neurosensibilité au Neurometer^{®} CPT

Un résumé des résultats correspondant à l'effet d'un extrait de racines de *Salvia miltiorrhiza* formulé à 2,5% comparé au placebo, sur la neurosensibilité cutanée évaluée à l'aide d'un Neurometer CPT sur des volontaires asiatiques, est présenté sur le Tableau 10.

**Tableau 10 Evolution du seuil de perception du courant entre J0 et J14 pour la formule placebo et l'extrait selon l'invention**

| | **Placebo** | **Extrait selon l'invention à 2,5%** |
|---|---|---|
| Neurosensibilité | 0 | -19 |

On constate qu'après 14 jours d'application, un extrait de racines de *Salvia miltiorrhiza* formulé à 2,5% induit une diminution significative de 19% de la neurosensibilité cutanée après stimulation des fibres nerveuses via un courant électrique de 250 Hz. Cet effet a été observé chez 65% des volontaires.

### Evaluation subjective

Les résultats de l'auto-évaluation réalisée par des volontaires caucasiens sont présentés dans le Tableau 11.

**Tableau 11. Auto-évaluation d'un extrait selon l'invention formulé à 2,5% après 14 jours d'applications biquotidiennes au niveau du visage en comparaison au placebo.**

| | **Cumul des réponses «plutôt d'accord» et «d'accord» (%)** | |
|---|---|---|
| | **Placebo** | **Extrait selon l'invention 2,5%** |
| **Avec ce soin ma peau est moins réactive** | 67 | 94 |
| **Ce soin est dédié aux peaux sensibles** | 67 | 94 |

On constate qu'après 14 jours d'application d'un extrait de racines de *Salvia miltiorrhiza* au niveau du visage :
- 94% des volontaires ont constaté que leur peau est moins réactive contre 67% pour le placebo (p = 0,0379) ;
- 94% des sujets s'accordent à dire que le soin est dédié aux peaux sensibles contre 67% des volontaires ayant utilisé le placebo (p = 0,0169).

### V. EFFET D'UN EXTRAIT SELON L'INVENTION SUR LA COMPOSANTE INFLAMMATOIRE

L'objectif de cette étude est d'évaluer *in vivo* l'influence d'un extrait de racines de *Salvia miltiorrhiza* formulé à 2,5% en émulsion sur la composante inflammatoire de la peau avant et après 28 jours d'applications biquotidiennes, en comparaison à une formule placebo.

La formule cosmétique contenant l'extrait est la suivante :

| | |
|---|---|
| Cetearyl ethylhexanoate (Lanol 1688, Seppic) | 5,0% |
| Extrait de Salvia miltiorrhiza selon l'invention (exemple 1) | 2,5% |
| Conservateurs | 1,0% |
| Carbomer (ULTREZ10, Noveon) | 0,5% |
| NaOH | Qsp pH 5 |
| Eau | qsp 100 |

Cet effet a été évalué en utilisant un modèle d'inflammation utilisant le leucotriène B4 (LTB4) dont l'application topique conduit à une inflammation locale de la peau en favorisant le recrutement de cellules inflammatoires leucocytaires. L'étude de la réponse inflammatoire de la peau suite à cette agression a été observée à l'aide d'un microscope confocal laser *in vivo* (Vivascope^{®}).

L'étude a été réalisée sur 17 volontaires sains caucasiens, de sexe féminin, d'âge moyen 44 ± 10 ans et présentant une peau normale au niveau des avant-bras intérieurs.

Un résumé des résultats correspondant au scorage de l'intensité de la réaction inflammatoire par des experts à partir des acquisitions effectuées par microscopie confocale laser *in vivo* est présenté sur le Tableau 12.

**Tableau 12 Effet d'un extrait selon l'invention formulé à 2,5% sur la réponse inflammatoire de la peau.**

| | **J0** | **J28** | **Variation/J0 (%)** |
|---|---|---|---|
| **Placebo** | 889 | 725 | -164% |
| **Extrait selon l'invention 2,5%** | 1022 | 618 | -404% |

On constate qu'après 14 jours d'application, un extrait de racines de *Salvia miltiorrhiza* formulé à 2,5% limite significativement la réponse inflammatoire locale de la peau face à une agression engendrée par une application topique de LTB4 (-240%, p = 0,0069). Cet effet a été observé chez 71% des volontaires.

### VI. EFFET D'UN EXTRAIT SELON L'INVENTION SUR LES PEAUX SENSIBILISEES PAR LE FROID

L'objectif de cette étude est d'évaluer *in vivo* l'influence d'un extrait de racines de *Salvia miltiorrhiza* formulé à 2,5% en émulsion sur la sensibilité cutanée engendrée par le froid sur des volontaires présentant une peau sensible.

La formule de la composition testée sur le visage et celle testée sur les mains est identique à celle du test présenté au point III.

Les études ont été réalisés avec les paramètres tels que décrits en suivants.

### A. Etude au niveau du visage

- Panel :
   20 volontaires sains caucasiens de sexe féminin, d'âge moyen 43 ± 10 ans ayant déclaré avoir une peau sensible selon les items du questionnaire SS-10 (Misery et al., Acta Derm Venereol, 2014), ayant réagi à la capsaïcine au cours d'un stinging test et présentant un seuil de tolérance au froid faible (mesure via le système TSA II).
- Modalités d'application :
   Applications biquotidiennes
- Méthodes :
   - étude du seuil de tolérance au froid mesuré au TSAII, au niveau du visage ;
   - étude de la rougeur cutanée après une exposition au froid sur photographie numérique ;
   - auto-évaluation de la performance perçue via un questionnaire d'évaluation subjective.

### B. Etude au niveau des mains

- Panel :
   13 volontaires sains caucasiens de sexe féminin, d'âge moyen 46 ± 11 ans ayant déclaré avoir une peau sensible selon les items du questionnaire SCC-10 (Misery et al., Acta Derm Venereol, 2014) et présentant un seuil de tolérance au froid faible au niveau des mains (mesure via le système TSA II).
- Modalités d'application :
   4 applications par jour (matin 8h, midi, après-midi 16h, au coucher)
- Méthodes :
   - étude du seuil de tolérance au froid mesuré au TSAII, au niveau des mains ;
   - auto-évaluation de la performance perçue via un questionnaire d'évaluation subjective.

Les résultats obtenus pour les différents tests sont présentés en suivant.

### Etude du seuil de tolérance au froid

Un résumé des résultats correspondant à l'effet d'un extrait de racines de *Salvia miltiorrhiza* formulé à 2,5% comparé au placebo, sur le seuil de tolérance au froid mesuré à l'aide du TSAII au niveau des joues et des mains, est présenté sur le Tableau 13.

**Tableau 13. Effet d'un extrait selon l'invention formulé à 2,5% sur le seuil de tolérance au froid.**

| | **Variation/Placebo (%)** |
|---|---|
| Visage | +7,5 |
| Mains | +15,5 |

On constate qu'après 14 jours d'application, un extrait de racines de *Salvia miltiorrhiza* réduit la sensibilité au froid de personnes présentant une peau sensible de 3°C sur le visage et de 4°C sur les mains. En effet, le seuil de tolérance au froid des volontaires est significativement augmenté à la fois au niveau du visage et des mains (respectivement +7,5%, p=0,0092 et +15,5%, p=0,0325). 70% d'entre eux ont présenté un effet positif sur le visage et 62% sur les mains.

### Etude de la rougeur cutanée après une exposition au froid

Un résumé des résultats correspondant à l'effet immédiat d'un extrait de racines de *Salvia miltiorrhiza* formulé à 2,5% comparé au placebo, sur le paramètre a* caractéristique de la rougeur de peau engendrée par un environnement froid, est présenté sur le Tableau 14.

**Tableau 14. Effet d'un extrait selon l'invention formulé à 2,5% en émulsion sur l'évolution de la rougeur cutanée après une exposition au froid de 15 minutes.**

| | **Augmentation de l'intensité de la rougeur cutanée après exposition au froid** |
|---|---|
| Placebo | 7E+05 |
| Extrait selon l'invention 2,5% | 4E+05 |
| Variation / placebo (%) | -43% |

Après application unique, un extrait de racines de *Salvia miltiorrhiza* formulé à 2,5% réduit la réactivité de la peau vis-à-vis du froid, caractérisée par une apparition de rougeurs cutanées (-43%, p = 0,0005). Cet effet a été observé chez 85% des volontaires.

### V I I. EVALUATION DE L'EFFET SUR LE CONFORT ET LA PROTECTION DE LA PEAU

### VII.1. Evaluation subjective par un panel caucasien

L'objectif de cette étude est d'évaluer *in vivo* l'influence d'un extrait de racines de *Salvia miltiorrhiza* formulé à 2,5% en émulsion sur la qualité de la peau (confort et protection) de volontaires présentant une peau sensible au niveau du visage et du corps.

Cet effet a été évalué par les volontaires en réalisant une auto-évaluation de la performance perçue via un questionnaire d'évaluation subjective en fin d'étude.

### A. Etude au niveau du visage

- Panel :
   20 volontaires sains caucasiens de sexe féminin, d'âge moyen 43 ± 10 ans ayant déclaré avoir une peau sensible selon les items du questionnaire SS-10 (Misery et al., Acta Derm Venereol, 2014), ayant réagi à la capsaïcine au cours d'un stinging test et présentant un seuil de tolérance au froid faible (mesure via le système TSA II).
- Modalités d'application :
   Applications biquotidiennes

### B. Etude au niveau des mains

- Panel :
   13 volontaires sains caucasiens de sexe féminin, d'âge moyen 46 ± 11 ans ayant déclaré avoir une peau sensible selon les items du questionnaire SCC-10 (Misery et al., Acta Derm Venereol, 2014) et présentant un seuil de tolérance au froid faible au niveau des mains (mesure via le système TSA II).
- Modalités d'application :
   4 applications par jour (matin 8h, midi, après-midi 16h, au coucher)

Les résultats obtenus pour les différents tests sont présentés en suivant.

### Etude au niveau du visage

Les résultats des questions fermées sont présentés dans le Tableau 15.

**Tableau 15. Auto-évaluation d'un extrait selon l'invention formulé à 2,5% après 14 jours d'applications biquotidiennes au niveau du visage en comparaison au placebo.**

| | **Cumul des réponses «plutôt d'accord» et «d'accord» (%)** | |
|---|---|---|
| | **Placebo** | **Extrait selon l'invention 2,5%** |
| **Ce soin protège ma peau des agressions extérieures** | 83 | 94 |
| **Ce soin diminue les sensations de tiraillements** | 61 | 83 |
| **Ce soin réduit l'inconfort cutané** | 67 | 94 |

On constate qu'après 14 jours d'application d'un extrait selon l'invention au niveau du visage :
- 94% des volontaires trouvent leur peau protégée des agressions extérieures (différence non significative) ;
- les sensations de tiraillements sont diminuées pour 83% des volontaires contre seulement 61% pour le placebo (p = 0,0387) ;
- l'inconfort cutané est réduit pour 94% d'entre elles contre seulement 67% pour le placebo (p = 0,0169).

### Etude au niveau des mains

Les résultats des questions fermées sont présentés dans le Tableau 16.

**Tableau 16. Auto-évaluation d'un extrait selon l'invention formulé à 2,5% après 14 jours d'applications au niveau des mains en comparaison au placebo.**

| | **Cumul des réponses «plutôt d'accord» et «d'accord» (%)** | |
|---|---|---|
| | **Placebo** | **Extrait selon l'invention 2,5%** |
| **Mains protégées** | 62 | 92 |
| **Soin nourrissant** | 77 | 100 |

On constate qu'après 14 jours d'applications d'un extrait selon l'invention au niveau des mains:
- 92% des volontaires ont constaté que leurs mains étaient protégées contre 62% pour le placebo (p = 0,0023) ;
- l'ensemble des sujets s'accorde à dire que le soin est nourrissant contre 77% des volontaires ayant utilisé le placebo (p<0,0001).

### VII.2. Evaluation subjective par un panel asiatique

L'objectif de ce test consommateurs est de comparer l'efficacité d'un soin contenant un extrait de racines de *Salvia miltiorrhiza* formulé à 2,5% à son placebo.

Cette étude a été réalisée sur 126 femmes vivant à Shanghai (Chine), d'âge compris entre 20 et 65 ans (âge moyen 37,3 +/- 9,6 ans), ayant déclaré avoir la peau sensible principalement à la pollution et éventuellement au froid ou aux changements de température et utilisatrices de soins visage pour peaux sensibles ou de produits revendiquant des effets anti-pollution.

Les volontaires ont été réparties en 2 groupes :
- groupe extrait selon l'invention : 63 femmes d'âge moyen 37 +/- 10 ans ;
- groupe placebo : 63 femmes d'âge moyen 37 +/- 10 ans.

Les évaluations ont été réalisées à la fin du 1^{er} jour de test ainsi qu'après 14 jours d'applications biquotidiennes à l'aide de questionnaires d'auto-évaluation.

Les résultats obtenus sont présentés en suivant.

### Résultats après la première journée d'application

Les résultats des questions fermées sont les suivants :

**Tableau 17. Perception des consommatrices après la première journée d'utilisation d'une formule contenant un extrait selon l'invention formulé à 2,5% en émulsion ou d'une formule placebo.**

| | **Cumul des réponses «D'accord » et** « **Tout à fait d'accord » (%)** | |
|---|---|---|
| | **Placebo** | **Extrait selon l'invention 2,5%** |
| Ma peau semble moins sensible | 77,8 | 88,9 |
| Les inconforts cutanés sont réduits | 84,1 | 92,1 |
| Ma peau me démange moins | 81,0 | 87,3 |

### Résultats après 14 jours d'utilisation

Les résultats des questions fermées sont les suivants :

**Tableau 18. Perception des consommatrices après 14 jours d'utilisation d'une formule contenant un extrait selon l'invention formulé à 2,5% en gel-émulsionné ou d'une formule placebo.**

| | **Consommatrices (%)** | |
|---|---|---|
| | **Placebo** | **Extrait selon l'invention 2,5%** |
| Ma peau semble hydratée durablement | 82,5 | 88,9 |
| Ma peau paraît plus souple | 84,1 | 85,7 |
| Ma peau semble nourrie | 90,5 | 95,2 |
| Ma peau semble moins sensible | 88,9 | 92,1 |
| Les inconforts cutanés sont réduits | 95,2 | 98,4 |
| Les rougeurs sont atténuées | 87,3 | 93,7 |
| Ma peau ne semble plus irritable | 82,5 | 88,9 |
| Ma peau est plus résistante aux changements de température | 85,7 | 87,3 |

On constate d'une manière globale que les femmes ayant utilisé la formule contenant un extrait de racines de *Salvia miltiorrhiza* ont attribué de meilleurs résultats aux différents items que celles ayant testé la formule placebo : il y a plus de femmes ayant observé une atténuation des rougeurs cutanées et s'accordant à dire que leur peau est moins sensible et moins irritable.

### VIII. ETUDE DE L'EFFET DE LA FRACTION POLYPHENOLIQUE D'UN EXTRAIT DE EXTRAIT DE RACINES DE SALVIA MILTIORRHIZA

L'objectif de cette étude est d'évaluer l'effet de la fraction polyphénolique d'un extrait de l'exemple 2.

100 mL d'extrait est traité avec du polyvinylpolypyrrolidone (5 g) afin d'éliminer les polyphénols. La solution obtenue est agitée pendant 2 heures à température ambiante puis filtrée. On obtient ainsi la Fraction A.

La Fraction A et l'extrait entier ont été analysés par chromatographie liquide ionique (système Dionex ICS 3000).

La quantité totale de polyphénols dans la Fraction A et dans l'extrait a été dosée par colorimétrie.

L'analyse des composés phénoliques a été effectuée par UPLC-UV-MS/MS.

Les analyses chromatographiques montrent que la fraction A est purifiée en sucres car elle contient 95% des sucres de l'extrait. Cette fraction a été épurée des polyphénols, car elle comporte seulement 11.5% des composés phénoliques de l'extrait.

L'essai de l'étude II.2. sur la sécrétion de PGE2 a été réalisée sur l'extrait et sur la Fraction A. Les résultats sont donnés dans le Tableau 19.

**Tableau 19. Capacité d'un extrait selon l'invention et de la Fraction A à limiter la sécrétion de PGE2 par des kératinocytes humains lors d'une inflammation induite par un polluant (PM).**

| | **Taux de PGE2 (pg/mg de protéines)** | **Taux de PGE2** / **témoin traité PM (%)** |
|---|---|---|
| **Kératinocytes normaux** | | |
| Témoin | 400 ±43 | |

| **Kératinocytes traités avec des PM** | | |
|---|---|---|
| Témoin | 594 ±71 | 20 |
| Extrait selon l'invention de l'exemple 2 à 0,050% | 412 ±71 | -94 |
| Fraction A à 0,05% | 588 ±63 | -3 |

On constate que la fraction A ne présente pas d'efficacité sur les PGE2 ce qui permet de montrer que les sucres ne confèrent pas cette efficacité à l'extrait.

Ce sont donc les composés phénoliques de l'actif, qui sont responsables de l'efficacité de l'extrait sur les PEG2.

### BIBLIOGRAPHIE

Boillat A, Alijevic O, Kellenberger S. Calcium entry via TRPV1 but not ASICs induces neuropeptide release from sensory neurons. Mol Cell Neurosci. 2014 Jul;61:13-22.

Draelos ZD. Sensitive skin: perceptions, evaluation, and treatment. Am J Contact Dermat Off J Am Contact Dermat Soc. 1997 Jun;8(2):67-78.

Duarte I, Silveira JEPS, Hafner M de FS, Toyota R, Pedroso DMM. Sensitive skin: review of an ascending concept. An Bras Dermatol. 2017;92(4):521-5.

Gouin O, L'Herondelle K, Lebonvallet N, Le Gall-lanotto C, Sakka M, Buhé V, et al. TRPV1 and TRPA1 in cutaneous neurogenic and chronic inflammation: pro-inflammatory response induced by their activation and their sensitization. Protein Cell. 2017 Mar 31;

Misery L, Loser K, Ständer S. Sensitive skin. J Eur Acad Dermatol Venereol JEADV. 2016 Feb;30 Suppl 1:2-8.

Pinto P, Rosado C, Parreirão C, Rodrigues LM. Is there any barrier impairment in sensitive skin?: a quantitative analysis of sensitive skin by mathematical modeling of transepidermal water loss desorption curves. Skin Res Technol Off J Int Soc Bioeng Skin ISBS Int Soc Digit Imaging Skin ISDIS Int Soc Skin Imaging ISSI. 2011 May;17(2):181-5.

Richters R, Falcone D, Uzunbajakava N, Verkruysse W, van Erp P, van de Kerkhof P. What is sensitive skin? A systematic literature review of objective measurements. Skin Pharmacol Physiol. 2015;28(2):75-83.

Richters RJH, Falcone D, Uzunbajakava NE, Varghese B, Caspers PJ, Puppels GJ, et al. Sensitive Skin: Assessment of the Skin Barrier Using Confocal Raman Microspectroscopy. Skin Pharmacol Physiol. 2017;30(1):1-12.

Saint-Martory C, Roguedas-Contios AM, Sibaud V, Degouy A, Schmitt AM, Misery L. Sensitive skin is not limited to the face. Br J Dermatol. 2008 Jan;158(1):130-3.

Tóth BI, Oláh A, Szöll 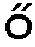 si AG, Bíró T. TRP channels in the skin. Br J Pharmacol. 2014 May;171(10):2568-81.

Vestergaard C, Hvid M, Johansen C, Kemp K, Deleuran B, Deleuran M. Inflammation-induced alterations in the skin barrier function: implications in atopic dermatitis. Chem Immunol Allergy. 2012;96:77-80.

## Revendications

1. Extrait de racines de de *Salvia miltiorrhiza* comprenant au moins 5% en poids de polyphénols par rapport au poids total de matière sèche de l'extrait pour son utilisation dans le traitement topique apaisant de la peau.

2. Extrait pour son utilisation selon la revendication 1, pour un traitement topique apaisant des peaux sensibles.

3. Extrait pour son utilisation selon l'une des précédentes revendications, pour réduire les réactions cosmétiques hypersensibles cutanées et/ou diminuer les sensations de tiraillement de la peau.

4. Extrait pour son utilisation selon l'une des revendications précédentes dans le traitement topique apaisant des peaux agressées par le froid et/ou par la pollution et/ou par des stress chimiques.

5. Extrait pour son utilisation selon la précédente revendication, pour :
- neutraliser l'hyperréactivité neuronale de la peau et/ou
- préserver la fonction barrière de la peau, et/ou
- limiter l'inflammation de la peau.

6. Extrait de racine de *Salvia miltiorrhiza* obtenu par une extraction à l'aide d'un solvant constitué d'eau et de butylène glycol d'origine végétale, comprenant au moins 5% en poids de polyphénols par rapport au poids total de matière sèche de l'extrait.

7. Extrait selon la précédente revendication, **caractérisé en ce qu'**il ne comprend pas de cryptotanshinone.

8. Extrait selon l'une des revendications 6 ou 7, **caractérisé en ce qu'**il comprend au moins 70% d'acides salvianoliques en poids par rapport au poids total des polyphénols présents dans l'extrait.

9. Extrait selon l'une des revendications 6 à 8 pour son utilisation dans le traitement topique apaisant de la peau.

10. Extrait selon l'une des revendications 6 à 8 pour son utilisation dans le traitement topique apaisant des peaux agressées par le froid et/ou par la pollution et/ou par des stress chimiques.

11. Composition cosmétique comprenant au moins 0,25% d'un extrait selon l'une des revendications 6 à 8 en poids total de la composition.

12. Composition selon la revendication 11, pour son utilisation en application topique sur la peau dans le traitement de la peau pour un effet apaisant.

13. Composition pour son utilisation selon la revendication 12, pour un effet apaisant des peaux sensibles.

14. Composition pour son utilisation selon la revendication 12 ou 13, pour un effet apaisant des peaux agressées par le froid et/ou par la pollution et/ou par des stress chimiques.

## Patentansprüche

1. Extrakt von *Salvia-miltiorrhiza-*Wurzeln, umfassend zu mindestens 5 Gew.-% Polyphenole, bezogen auf das Gesamtgewicht der Trockenmasse des Extrakts, für die Verwendung bei der beruhigenden topischen Behandlung der Haut.

2. Extrakt für die Verwendung nach Anspruch 1 für eine beruhigende topische Behandlung empfindlicher Haut.

3. Extrakt für die Verwendung nach einem der vorstehenden Ansprüche zum Reduzieren kutaner überempfindlicher kosmetischer Reaktionen und/oder zum Vermindern der Spannungsgefühle der Haut.

4. Extrakt für die Verwendung nach einem der vorstehenden Ansprüche bei der beruhigenden topischen Behandlung von durch Kälte und/oder durch Verunreinigung und/oder durch chemische Belastungen angegriffene Haut.

5. Extrakt für die Verwendung nach dem vorstehenden Anspruch, zum:
- Neutralisieren der neuronalen Hyperreaktivität der Haut und/oder
- Erhalten der Barrierefunktion der Haut und/oder
- Einschränken der Entzündung der Haut.

6. Extrakt von der *Salvia miltiorrhiza-Wurzel,* der durch eine Extraktion mittels eines Lösungsmittels erhalten wird, das aus Wasser und Butylenglykol pflanzlichen Ursprungs, umfassend zu mindestens 5 Gew.-% Polyphenole, bezogen auf das Gesamtgewicht der Trockenmasse des Extrakts, besteht.

7. Extrakt nach dem vorstehenden Anspruch, **dadurch gekennzeichnet, dass** er kein Cryptotanshinon umfasst.

8. Extrakt nach einem der Ansprüche 6 oder 7, **dadurch gekennzeichnet, dass** er zu mindestens 70 Gew.-% Salvianolsäuren, bezogen auf das Gesamtgewicht der in dem Extrakt vorhandenen Polyphenole, umfasst.

9. Extrakt nach einem der Ansprüche 6 bis 8 für die Verwendung bei der beruhigenden topischen Behandlung der Haut.

10. Extrakt nach einem der Ansprüche 6 bis 8 für die Verwendung bei der beruhigenden topischen Behandlung von durch Kälte und/oder durch Verunreinigung und/oder durch chemische Belastungen angegriffene Haut.

11. Kosmetische Zusammensetzung, umfassend zu mindestens 0,25 Gew.-% des Gesamtgewichts der Zusammensetzung einen Extrakt nach einem der Ansprüche 6 bis 8.

12. Zusammensetzung nach Anspruch 11 für die Verwendung bei einer topischen Anwendung auf der Haut bei der Behandlung der Haut für eine beruhigende Wirkung.

13. Zusammensetzung für die Verwendung nach Anspruch 12 für eine beruhigende Wirkung auf empfindliche Haut.

14. Zusammensetzung für die Verwendung nach Anspruch 12 oder 13 für eine beruhigende Wirkung auf durch Kälte und/oder durch Verunreinigung und/oder durch chemische Belastungen angegriffene Haut.

## Claims

1. Root extract from *Salvia miltiorrhiza* comprising at least 5% by weight of polyphenols based on the total weight of dry matter of the extract, for use in the topical soothing treatment of the skin.

2. Extract for use according to claim 1, for topical soothing treatment of sensitive skin.

3. Extract for use according to either of the preceding claims, for reducing hypersensitive skin reactions to cosmetics and/or for reducing the feeling of skin tightness.

4. Extract for use according to any of the preceding claims in the topical soothing treatment of skin damaged by the effects of cold and/or pollution and/or chemical stress.

5. Extract for use according to the preceding claim, for:
- neutralizing neuronal hyperreactivity of the skin and/or
- preserving the barrier function of the skin, and/or
- limiting inflammation of the skin.

6. Root extract from *Salvia miltiorrhiza* obtained by extraction by means of a solvent consisting of water and butylene glycol of vegetable origin, comprising at least 5% by weight of polyphenols based on the total weight of dry matter of the extract.

7. Extract according to the preceding claim, **characterized in that** it does not include cryptotanshinone.

8. Extract according to either of claims 6 or 7, **characterized in that** it comprises at least 70% by weight of salvianolic acids based on the total weight of polyphenols in the extract.

9. Extract according to any of claims 6 to 8 for use in the topical soothing treatment of the skin.

10. Extract according to any of claims 6 to 8 for use in the topical soothing treatment of skin damaged by the effects of cold and/or pollution and/or chemical stress.

11. Cosmetic composition comprising at least 0.25% by weight of an extract according to any of claims 6 to 8, based on the total weight of the composition.

12. Composition according to claim 11 for use in topical application to the skin for treating the skin with a soothing effect.

13. Composition for use according to claim 12, for providing a soothing effect to sensitive skin.

14. Composition for use according to claim 12 or 13, for providing a soothing effect to skin damaged by the effects of cold and/or pollution and/or chemical stress.
